# EUROPEAN PATENT APPLICATION

(11) **EP 4 730 339 A1**
(43) Date of publication of application: **22.04.2026**
(21) Application number: 24383141.9
(22) Date of filing: 17.10.2024
(51) Int. Cl.: G16B 25/20, G16B 30/10, G16B 40/00

(54) **METHOD FOR TARGET METAGENOMIC SEQUENCING OF MULTIPLE MICROBIAL GENE FAMILIES WITH FULL PHYLOGENETIC COVERAGE IN A SINGLE EXPERIMENT**

(71) Applicant: Consejo Superior de Investigaciones Científicas (CSIC), 28006 Madrid (ES); Universidad Politécnica de Madrid, 28040 Madrid (ES)
(72) Inventor: HUERTA CEPAS, Jaime, 28006 Madrid (ES); GONZALEZ BODÍ, Sara, 28006 Madrid (ES); PÉREZ CANTALAPIEDRA, Carlos, 28040 Madrid (ES); SANCHIS LÓPEZ, Claudia, 28040 Madrid (ES); POKORNY MONTERO, Cristina Isabel, 28040 Madrid (ES)
(74) Representative: Hoffmann Eitle

(57) **Abstract**

The vast majority of prokaryotic microorganisms remains uncultured and are not abundant enough to be effectively studied using metagenomic sequencing methods. Here, we present a novel PCR-free target capture sequencing approach that enables targeting specific genes or functions with full phylogenetic coverage in a single sequencing experiment, proving also a higher sensitivity and precision than regular shotgun and amplicon-based approaches.

## Description

### TECHNICAL FIELD

The present invention refers to a method for targeting, in a single sequencing experiment of an environmental or biological sample, multiple microbial gene families, providing full phylogenetic coverage, high-sensitivity to detect low-abundance organisms and strain-level precision in the microorganisms identification.

### BACKGROUND ART

Microorganisms dominate the earth and have shaped the planet as we know it. However, despite their importance, our current knowledge of the microbial world remains very limited. Today, we know that microbial biodiversity, both at the phylogenetic and functional levels, is dominated by a large number of species that we have barely started to understand and even detect. The vast majority of them have not been cultured in the lab, and many more are not abundant enough to be studied with current genomic approaches.

This huge fraction of elusive microbes, usually referred to as the rare biosphere, is considered of utmost importance to understand and predict microbiome responses to environmental change, especially in highly biodiverse settings such as free-living habitats. It is known that the rare biosphere harbors keystone species that, even at very low abundance, can modulate nitrogen fixation, phosphorus cycling, and sulfur and carbon flows. The rare members of microbial communities are also hypothesized to represent a large reservoir of xenobiotic metabolism (i.e., involved in biodegradation), therefore determining the resilience of microbial ecosystems to pollutants, pesticides, and antibiotics. Likewise, it is believed that latent organisms in the rare biosphere can heavily affect the tolerance of natural habitats to climate change.

However, uncovering low abundant microorganisms using metagenomics and metataxonomic techniques is extremely challenging. Amplicon-based sequencing of marker genes (e.g., 16S, 18S, ITS) provides predictions with limited phylogenetic resolution, entails important technical biases associated with their amplification, produces inaccurate abundance profiles due to copy-number variation, and misses the detection of phylogenetically distant unknown lineages (Bahram et al. 2019). Full length 16S sequencing mitigates some of these problems, achieving nearly species-level resolution, but it cannot reach strain resolution and does not allow for phylogenomic approaches. Similarly, PCR amplification of universal single-copy markers has been previously tested as an unbiased and higher resolution alternative to 16S and whole-genome sequencing. However, PCR approaches are only feasible for targeting specific genes from specific species, therefore incapable of providing wide phylogenetic coverage.

On the other hand, shotgun metagenomic sequencing offers a comprehensive view of the genomes present across entire microbial communities, but the sequenced data is typically saturated with the genetic material of the most-abundant members. To date, even the highest-depth sequencing efforts in metagenomics can only provide random glimpses of the genetic variability present in the rare biosphere.

In summary, there are currently no methods capable of uncovering all the genetic variability of one or multiple specific gene families in a metagenomic sample, with full phylogenetic coverage and covering both high and low-abundance microorganisms present in a single experiment.

In the present invention, we propose a novel PCR-free target metagenomics sequencing method based on the use of in-solution DNA hybridization techniques, prior to shotgun sequencing, to capture and enrich all DNA fragments in a sample corresponding to the desired pool of gene families. In-solution hybridization strategies are known to deliver much higher resolution in the detection of novel genetic variants than traditional PCR and Sanger sequencing, but its use for taxonomic and functional profiling of metagenomic samples is currently limited by the high number of sequence probes needed to cover all microbial biodiversity at once, and the lack of sufficiently accurate probes to target particular gene families across all microorganisms.

A key component of the invention, therefore, lies in our methodology to design custom enrichment panels composed of an optimal set of nucleotide sequence probes that 1) provide high sensitivity and precisión in targeting the desired gene families, reaching DNA material in very low abundance 2) cover all known sequence variability of the desired pool of gene families 3) cover potential sequence diversity from unknown species 4) can be manufactured in a single capture hybridization panel using current technology. One example of our custom capture panel uses a set of 580,369 probes optimized to cover all known biodiversity of ten universally conserved marker genes commonly used for high-precision taxonomic profiling, allowing to identify low-abundance species across the entire bacterial phylogeny and reach strain level resolution in the taxonomic identification. By validating our method on 57 samples from different habitats, including human gut microbiome, soil and extreme environments, we show that the method provide unprecedented taxonomic coverage and phylogenetic resolution on samples of diverse environments, outperforming standard shotgun metagenomic sequencing and amplicon-based methods.

### BRIEF DESCRIPTION OF THE FIGURE

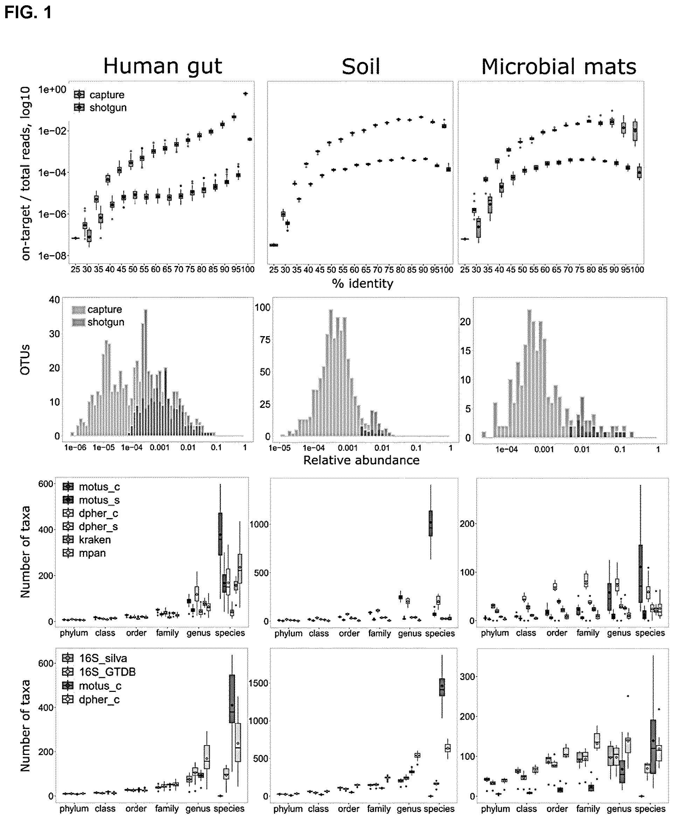

Figure 1. Capture efficiency and taxonomic profiling from DNA capture of 10 single-copy universal marker genes in samples from human gut (1st column), soil (2nd column) and microbial mats (3rd column). A) Proportion of on-target reads (on-target reads / total clean reads, log10 scale), arranged by intervals of % of identity computed from alignments of on-target reads to the original MGs used to design the probes, for capture (red) and shotgun (blue) data. B) mOTUs (-g3) relative abundance capture vs shotgun, with the same number of 2x100 pair reads (standardized set) for each capture (red) / shotgun (blue) sample pair (standardized set). C) Number of observed taxa across all taxonomic ranks with mOTUs (-g3; "motus_c", "motus_s"; capture and shotgun data, respectively)), DECIPHER (dpher_c, dpher_s; capture and shotgun data, respectively), MetaPhlAn 4 ("mpan") and Kraken2 ("kraken"), with the same number of 2x100 pair reads (standardized set) for each capture / shotgun sample pair (standardized set). D) Number of observed taxa across all taxonomic ranks with 16S (Silva DB; "16S_silva"), 16S (GTDB DB, "16S_GTDB"), mOTUs (capture data, -g 3, "motus_c"), DECIPHER (capture data, "dpher_c").

### BRIEF DESCRIPTION OF THE INVENTION

The vast majority of prokaryotic microorganisms remains uncultured and are not abundant enough to be effectively studied using metagenomic sequencing methods. Here, we present a novel PCR-free target capture sequencing approach that enables targeting specific genes or functions with full phylogenetic coverage in a single sequencing experiment, proving also a higher sensitivity and precision than regular shotgun and amplicon-based approaches. We demonstrate the validity and practical application of our approach by using the method to design a capture-kit panel targeting ten universally-conserved marker genes, enabling the accurate identification of low abundant microorganisms with broad phylogenetic coverage. When applied on human gut, soil and extreme environments samples, our method identifies up to 5 times more species than conventional shotgun or 16S sequencing, achieving strain level resolution and enabling population metagenomic analysis. Furthermore, it unveils a large amount of unknown low-abundance microorganisms present in the rare microbial biosphere.

Therefore, one aspect of the present invention refers to an *in vitro* method for providing a collection of sequencing probes that enable targeting, preferably in a single sequencing experiment, one or various gene families or functions, said method comprising the steps of:
a. Step 1. Gene family selection: Compiling a list of marker gene families or functional orthologous groups (OG) by, preferably, identifying genes from a database within or associated to a specified group of molecular interactions and reaction networks;
b. Step 2 Data mining: Identifying and providing from one or more databases, a data corpus or dataset of nucleotide sequences having a percentage of nucleotide identity with the genes and OGs identified in step 1 of at least 50%, 60%, 70%, 80%, 90%, 95%, 99% or more;
c. Step 3. Design of capture-kits: Analyzing the data corpus or dataset provided in Step 2 in order to reduce its dimensionality to make it accessible for a single targeted sequencing experiment, preferably while maintaining the full phylogenetic coverage and high sensibility.
d. Providing the design of the panel or collection of sequencing probes that enable targeting, preferably in a single sequencing experiment, one or various gene families or functions selected in Step 1.
e. Optionally, manufacturing or producing the panel of step d.

In a preferred embodiment, in Step 2 the data corpus of nucleotide sequences is provided by retrieving for each of the selected OGs of step 1, the sequences from (i) global genomic repositories and (ii) public metagenomic datasets.

In another preferred embodiment, Step 3 is carried out by a method comprising the following steps i) to iv) for each targeted OG (tOG) included in a kit:
i. Decomposing into non-overlapping chunks of from 10 - 200 pair bases, preferably from 50 to 150 pair bases, more preferably 80 or 120 pair bases, all or part of the nucleotide sequences comprising the dataset obtained in Step 2 for each of the tOG, herein referred to as probes.
ii. Aligning the nucleotide sequences comprising the dataset obtained in step 2 into a multiple sequence alignment (MSA), where the conservation level of each aligned column is calculated.
iii. The multiple sequence alignment produced is used to generate a phylogenetic tree of all or part of the non-redundant sequences.
iv. All or part of the generated probes in step i) are compared in an all-against-all fashion to calculate their pairwise sequence similarity, computed as the % of nucleotides identities observed between two probes and provide an all-against-all probe comparison matrix.
wherein the method further comprises the following steps:
v. Using the all-against-all probe comparison matrix of step iv to generate clusters of probes at the NSI (Nucleotide Sequence Identity) threshold, wherein the NSI threshold is selected from one or more values in the range of from 30% to 100%.
vi. Mapping all or part of the probe clusters of step v back to the same original genomic and metagenomic databases used in Step 2 using BLASTN searches.
vii. Analyzing to compute the ratio of positive matches (i.e. hits against a gene belonging to the expected OG) over negative matches (i.e. hits against a gene belonging to an unwanted OG) of the hits produced in step vi.
viii. Discarding all probe clusters with a ratio of positive matches lower than 1.0 (i.e. probes producing at least one mismatch) from the set.
ix. Mapping all or part of the remaining selected probe clusters to the MSA generated in step ii), preferably by recording the exact position of matching residues of the original probe on the MSA.
x. Discarding probes matching columns of the alignment produced in ii with a residue conservation score, preferably of below 0.75.
xi. Mapping back the final set of selected probes to the original sequences selected in Step 2, preferably by using BLASTN, and preferably calculating: number of original sequences recovered by the probes (RecovT), the average length of the original sequence covered by the probe-selection (RecovLen) and the phylogenetic coveraged based on the the generated in substep C (PhyloC)

Preferably, in connection to the above embodiment, for each tOG the total number of probes selected (Nprobes), together with their RecovT, RecovL and PhyloC values obtained for each clustering experiment under each NSI are automatically evaluated to select the set of probes that maximizes RecovT, RecovL and PhyloC, while minimizing NProbes.

More preferably, the set of probes that maximizes RecovT, RecovL and PhyloC, while minimizing Nprobes, for each tOG, is combined into a single dataset, and providing the final design of the panel. Still, more preferably, the method further comprises the manufacturing or production of the said panel of probes.

A further aspect of the invention refers to a panel obtained or manufactured or produced by the above method, wherein the probes are polynucleotides, preferably DNA or RNA, probes. Preferably, this panel is used for capturing and target enrichment assays to identify organisms, either known or unknown organisms.

Further aspects of the present invention refer to a kit comprising the panel obtained or manufactured or produced by the above method.

Said kit or panel can be useful for:
a. Hybridizing custom capture-kits with environmental DNA samples prior to sequencing to specifically select the targeted genes
b. Shotgun sequencing of the enriched DNA sample

### DEFINITIONS

As used herein, "functional orthologous groups (OG)" shall be understood as a comprehensive group of nucleotide sequences representing genes from all known species with a common evolutionary origin and molecular and cellular function.

As used herein "marker gene families", shall be understood as a set of functional orthologous groups (OGs) that are representative for a given microbial function or characteristic. For instance, phylogenetic marker gene families refer to OGs that are particularly suitable to establish the phylogenetic position and evolutionary relationships among species, as they are composed of highly coserved genes involved in the molecular functions present in all living organisms. Similarly, marker genes families for the microbial function associated with nitrogen fixation would refer to one or various OGs where genes encode for molecular functions highly specific for the process of nitrogen fixation.

As used herein, "group of molecular functions" shall be understood as the molecular activities performed by a genes.

As used herein, "full phylogenetic coverage" shall be understood as the fact of sequencing, detecting or using genes from all microbial species known to date.

As used herein, when referring to "the conservation level of each column is calculated" shall be understood as the assessment of the probability that an specific nucleotide base (i.e. A, C, T or G) is the same in all genes from the same OG.

As used herein, when referring to "decomposing into non-overlapping chunks" shall be understood as the fragmentation of long DNA strings into smaller consecutive strings.

As used herein, "a phylogenetic tree" shall be understood as the tree-like data structure that represents the evolurionary relationships among all genes/sequences. I

As used herein, "non-redundant sequences" shall be understood as unique sequences that differ from the rest in at least one nucleotide.

As used herein, "compared in an all-against-all fashion" shall be understood as the comparison of all possible pairs of sequences.

As used herein, "calculate their pairwise sequence similarity" shall be understood as the calculation of identical nucleotide positions between two sequences, expressed as percentage of the total length of the shortest sequence.

As used herein, "all-against-all probe comparison matrix" shall be understood as the matrix of nucleotide identity values obtained for all pairwise sequence comparisons.

As used herein, "clusters of probes at the NSI threshold" shall be understood as the process of grouping probes based on their nucleotide sequence identity (NSI). For instance, clustering at NSI=80% would mean that all probes with a 80% NSI or higher are grouped together into a single cluster.

As used herein, "mapping back to the same original genomic and metagenomic databases using BLASTN searches" shall be understood as the comparison of nucleotide probe sequences against a sequence database using the blast-n software.

As used herein, the phrase "maximizes RecovT, RecovL and PhyloC, while minimizing Nprobes, for each tOG" shall be understood as the automated process by which the methods selectes the set of probes that contains the least numnber of probes, while maintaining nearly full phylogenetic coverage and high sensitivity and precision.

### DESCRIPTION

As indicated previously, in the present invention, we present a novel PCR-free target metagenomics sequencing approach based on a custom capture enrichment panel covering the entire prokaryotic phylogeny. Such custom capture enrichment panel (comprising capture sequence probes that cover an entire prokaryotic phylogeny) can be obtained by an *in vitro* method in accordance with the first aspect of the present invention,

In the present invention, we postulate that full phylogenetic coverage for many key gene families could be implemented in relatively inexpensive capture kits, increasing the precision and coverage of current functional and taxonomic profiling methodologies. This is supported by (i) the great level of customization presently available for the design of sequence probes, (ii) the available size range of current capture panels (admitting up to hundreds of thousands of different probes), and (iii) the estimated extent of the genetic variability for most important marker genes in ecology.

Although there is no record in the literature for such a broad implementation, our tests performed during the last years indicate that high-depth exploration of key functional and phylogenetic markers genes from rare genomes are technically feasible, provided the manufacturing of capture kits and target enrichment protocols are combined with phylogenetically-driven design of target probes. In this sense, in the present invention we provide a study showing that, indeed, a single capture kit based on ten phylogenetic marker gene families suffices to cover all known prokaryotic diversity (~12,000 references prokaryotic species), detecting microbial species at levels of abundance two orders of magnitude below of what regular shotgun sequencing can reach (Figure 1), and uncovering three times as much biodiversity (Figure 1).

Furthermore, our results uncover hundreds of unclassified on-target sequences lacking close phylogenetic relatives, which might represent detections for never-seen low abundance organisms. This is a desirable effect derived from the fact that template probes can be designed to capture, not only the diversity of all known genes, but also a certain degree (up to 30%) of unknown genetic variability. The above results were obtained in our laboratory by testing a pilot custom capture kit and applying it on a variety of environmental and human gut samples, using the same sequencing depth for both shotgun and capture plus shotgun experiments. These results prove that it is technically possible to design broad hypothesis-driven capture panels (i.e., spanning vast phylogenetic distances) targeting, not only phylogenetic marker genes, but also dozens of ecologically relevant gene functions.

In particular, the procedure for designing and manufacturing broad hypothesis-driven capture panels provided by the present invention comprises the following general steps:

### - Step 1. Gene family selection:

In order to maximize the functional coverage, a list of marker gene families needs to be compiled. That is, there is a need to identify genes from a database within or associated to a specified group of molecular interactions and reaction networks.

For example, to design and manufacture a kit targeting the precise identification of all microorganisms present in a metagenomics sample, we used a set of universal, single copy phylogenetic markers genes. Coverserly, to unveil all the biodiversity of metabolic genes involved in the degradation of soil pollutants, we would target maker enzymes and transporters related to the biodegradation of drugs (including antibiotics), dioxins, hydrocarbures, benzones, and other pollutants such as heavy metal resistance genes. Thus, for this first step, we shall depart from the already known sequences associated with each of the functional descriptions considered as a functional marker. This is typically referred to as a functional orthologous group (OG).

### - Step 2 Data mining to maximize phylogenetic coverage:

In this step, we shall identify from one or more databases nucleotide sequences having a percentage of identity with the genes and OGs identified in step 1 of at least 60%, 70%, 80%, 90%, 95%, 99% or more.

For our specific examples, for each of the selected OGs, we will pull all available sequences from (i) global genomic repositories and (ii) public metagenomic datasets. By mining both genomic and metagenomic variability, we aim at ensuring that subsequent enrichment experiments are not biased towards data from cultivated taxa or specific lineages. The resulting corpus of genomic and metagenomics sequences matching each of the targeted OG represents all known gene sequence variability across all cultivated and uncultivated microbial organisms. Due to the volume and extent of such corpus of information, it is currently unfeasible to target all such variability at once in a single sequencing experiment

### Step 3. Design of capture-kits:

In this step, we analyze the data corpus produced in Step 2 in order to reduce its dimensionality to make it accessible for a single targeted sequencing experiment, while maintaining the same phylogenetic coverage and sensibility and improving its precision. This involved a series of in-vitro computational analysis and actions as detailed in the following pseudocode:
Loop 1: For each targeted OG (tOG) included in a kit:
   A. All nucleotide sequences comprising the dataset obtained in step 2 for the tOG are decomposed into non-overlapping chunks of 80 pair bases, herein referred to as probes.
   B. All nucleotide sequences comprising the dataset obtained in step 2 are aligned into a multiple sequence alignment, where the conservation level of each column is calculated.
   C. The multiple sequence alignment produced is used to generate a phylogenetic tree of all non-redundant sequences.
   D. All the generated probes in substep A are compared in an all-against-all fashion to calculate their pairwise sequence similarity, computed as the % of nucleotides identities observed between two probes.
Loop 2: For each of the possible nucleotide sequence identity (NSI) in 100%, 90%, 80%, 70%:
   E. The all-against-all probe comparison matrix is then used to generate clusters of probes at the NSI threshold. Here, we intend to produce a reduced set of probes compatible with the current sensitivity of capture protocols, which varies from 100% to 30% NSI. This is possible because, for in-solution hybridization capture kits, capture probes can be used in the same way as degenerate primers can, albeit providing capture ranges of up to 30% divergence from the original template
   F. All probe clusters are mapped back to the same original genomic and metagenomic databases used in step 2 using BLASTN searches.
   G. The significant hits produced in substep F are analyzed to compute the ratio of positive matches (i.e. hits against a gene belonging to the expected OG) over negative matches (i.e. hits against a gene belonging to an unwanted OG). Notice that, although all probes were generated from targeted OG genes, many might belong to promiscuous gene regions that exist in many other OGs, potentially dismissing the precision of the targeted OG detections.
   H. All probe clusters with a ratio of positive matches lower than 1.0 (i.e. probes producing at least one mismatch) are discarded from the set, therefore keeping only highly specific probes of the tOG and reducing potential off-target hybridization in the kit.
   I. All the remaining selected probe clusters are then mapped to the MSA generated in substep B), recording the exact position of matching residues of the original probe on the MSA.
   J. Probes matching columns of the alignment produced in B with a residue conservation score below 0.75 are discarded, keeping only specific probes that are also largely universal for the tOGs (i.e. discarding marginal probes).
   K. The final set of selected probes is then mapped back to the original sequences selected in Step 2 using BLASTN, calculating: number of original sequences recovered by the probes (RecovT), the average length of the original sequence covered by the probe-selection (RecovLen) and the phylogenetic coveraged based on the the generated in substep C (PhyloC)

The total number of probes selected (Nprobes), together with their RecovT, RecovL and PhyloC values obtained for each clustering experiment under each NSI are automatically evaluated to select the set of probes that maximizes RecovT, RecovL and PhyloC, while minimizing NProbes. Finally, the best probe set for each tOG is combined into a single dataset, which is considered the final design of the panel.

Therefore, in a first aspect of the invention, we herein provide an *in vitro* method for providing a collection of highly optimized sequencing probes that enable targeting, preferably in a single sequencing experiment, one or various gene families or functions, providing full phylogenetic coverage and higher sensitivity and precision than current methods based on shotgun and amplicon based sequencing, said method comprising the steps of:

### - Step 1. Gene family selection:

Compiling a list of marker gene families or functional orthologous groups (OG) by, preferably, identifying genes from a database within or associated to a specified group of molecular interactions and reaction networks.

Thus, for this first step, we shall depart from the already known sequences associated with each of the functional descriptions considered as a functional marker.

### - Step 2 Data mining to maximize phylogenetic coverage:

Identifying and providing from one or more databases, nucleotide sequences having a percentage of identity with the genes and OGs identified in step 1 of at least 60%, 70%, 80%, 90%, 95%, 99% or more. Preferably, for each of the selected OGs of step 1, retrieving all available sequences from (i) global genomic repositories and (ii) public metagenomic datasets. By mining both genomic and metagenomic variability, we aim at ensuring that subsequent enrichment experiments are not biased towards data from cultivated taxa or specific lineages. The resulting corpus of genomic and metagenomics sequences matching each of the targeted OG represents all known gene sequence variability across all cultivated and uncultivated microbial organisms. As already indicated due to the volume and extent of such corpus of information, it is currently unfeasible to target all such variability at once in a single sequencing experiment

### Step 3. Design of capture-kits:

Analyzing the data corpus produced in Step 2 in order to reduce its dimensionality to make it accessible for a single targeted sequencing experiment, while maintaining the same phylogenetic coverage and sensibility and improving its precision. Preferably, by carrying out the following steps:
Substep 1: For each targeted OG (tOG) included in a kit:
   A. Decomposing into non-overlapping chunks of from 10 - 200 pair bases, preferably from 50 to 100 pair bases, more preferably about 80 pair bases, all or part of the nucleotide sequences comprising the dataset obtained in step 2 for each of the tOG, herein referred to as probes.
   B. Aligning the nucleotide sequences comprising the dataset obtained in step 2 into a multiple sequence alignment (MSA), where the conservation level of each column is calculated.
   C. The multiple sequence alignment produced is used to generate a phylogenetic tree of all or part of the non-redundant sequences.
   D. All the generated probes in substep A are compared in an all-against-all fashion to calculate their pairwise sequence similarity, computed as the % of nucleotides identities observed between two probes and provide an all-against-all probe comparison matrix.
Substep 2:
   E. Using the all-against-all probe comparison matrix of step D to generate clusters of probes at the NSI threshold. Here, we intend to produce a reduced set of probes compatible with the current sensitivity of capture protocols, which varies from 100% to 30% NSI. This is possible because, for in-solution hybridization capture kits, capture probes can be used in the same way as degenerate primers can, albeit providing capture ranges of up to 30% divergence from the original template
   F. Mapping all or part of the probe clusters of step E back to the same original genomic and metagenomic databases used in step 2 using BLASTN searches.
   G. Analyzing to compute the ratio of positive matches (i.e. hits against a gene belonging to the expected OG) over negative matches (i.e. hits against a gene belonging to an unwanted OG) of the hits produced in step F. Notice that, although all probes were generated from targeted OG genes, many might belong to promiscuous gene regions that exist in many other OGs, potentially dismissing the precision of the targeted OG detections.
   H. Discarding all probe clusters with a ratio of positive matches lower than 1.0 (i.e. probes producing at least one mismatch) from the set. This step thus provides keeping only highly specific probes of the tOG and reducing potential off-target hybridization in the kit.
   I. Mapping all or part of the remaining selected probe clusters to the MSA generated in substep B), preferably by recording the exact position of matching residues of the original probe on the MSA.
   J. Discarding probes matching columns of the alignment produced in B with a residue conservation score, preferably of below 0.75. This step provides keeping only specific probes that are also largely universal for the tOGs (i.e. discarding marginal probes).
   K. Mapping back the final set of selected probes to the original sequences selected in Step 2, preferably by using BLASTN, and preferably calculating: number of original sequences recovered by the probes (RecovT), the average length of the original sequence covered by the probe-selection (RecovLen) and the phylogenetic coveraged based on the the generated in substep C (PhyloC)

In a preferred embodiment, the total number of probes selected (Nprobes), together with their RecovT, RecovL and PhyloC values obtained for each clustering experiment under each NSI are automatically evaluated to select the set of probes that maximizes RecovT, RecovL and PhyloC, while minimizing NProbes. Finally, the best probe set for each tOG is combined into a single dataset, thus providing the design of the panel.

In one embodiment of the first aspect of the invention, the databases of step 2 (Data mining) comprise genomic and metagenomic databases.

A second aspect of the invention refers to a set of oligonucleotides or probes identified or produced in accordance with the first aspect of the invention, or in accordance with any of the preferred embodiments of the first aspect of the invention. Preferably, the oligonucleotides or probes are DNA or RNA oligonucleotides or probes.

A third aspect of the invention refers to the in vitro use of a set of oligonucleotides or probes identified or produced in accordance with the first aspect of the invention, or in accordance with any of the preferred embodiments of the first aspect of the invention, in capture and target enrichment assays to identify known or unknown organisms. Kits comprising the above mentioned set of oligonucleotides or probes identified or produced in accordance with the first aspect of the invention also form part of the present invention.

The following examples serve to illustrate the present invention but do not limit the same.

### EXAMPLES

### Optimized target capture sequencing of ten maker genes provide full phylogenetic coverage and strain resolution in a single experiment:

Using the methodology described in previous steps, we designed a custom set of DNA oligonucleotide probes targeting ten universally-conserved marker genes (MGs) suitable for high resolution phylogenetic analysis and taxonomic profiling of metagenomics samples and commonly employed by bioinformatic software (mOTUs, https://www.nature.com/articles/s41467-019-08844-4). Each of the ten genes selected represent a targeted orthologous groups (tOG) in substeps A-K. Probes were generated from the coding nucleotide sequences of the ten marker genes extracted from reference genomic and metagenomic databases, comprising a total of 7,726 prokaryotic genomes and metagenomic assemble genomes and providing coverage for nearly full bacterial and archeal biodiversity.

From the total number of probes obtained in step A (~3 million), the optimization substeps generated ~500,000 total selected probes distributed along the ten tOGs. Next, we manufactured a custom hybridization capture kit using the 500k selected probes and performed enrichment experiments on various environmental samples. In particular, we analyzed the sensitivity, phylogenetic coverage and precision of the generated kit on 57 metagenomic samples from human gut (32 samples), soil (16 samples), and microbial mats (9 samples), for which the standard shotgun metagenomics and 16 amplicon sequencing approaches were also performed for comparison reasons.

Capture experiments were successful for all samples, achieving an on-target enrichment rate (i.e. total reads matching probes) from 70% (human gut) to 20% (other natural environments).

### High sensitivity and phylogenetic coverage: Accurate detection of low abundance species

To test the sensitivity of produced capture kit design, we estimated the taxonomic profile of all samples using the mOTUs tool, which is considered an standard method in the field and is optimized to obtain the presence and relative abundance of microorganisms in a metagenomic samples using the same ten marker genes used in our capture panel.

On average, our target capture resulted in the detection of up to 14-fold more mOTUs species per sequencing depth unit than regular shotgun across all samples (Figure 1B). This increase was consistent regardless of the stringency level used for species calls in the mOTUs tool. Most species detected in the shotgun data were also detected in the capture data (% average match), and their relative abundances were strongly correlated, demonstrating that the capture method increased detection without biasing abundance estimates (Figure 1). In particular, capture detections were significantly enriched in the low abundance range (Figure 1 histograms), highlighting a high level of sensitivity currently unreachable by either shotgun or amplicon methods. The number of mOTUs-based species detections derived from target capture data increased not only compared to mOTUs shotgun results, but also compared to the number of species detected by other standard tools such as MetaPhlAn4 and and Kraken2.

### High precision: Strain resolution level in the identification of microorganisms in the samples

Levering on the high sequencing depth obtained for the targeted makers genes, we evaluated whether fine-grained taxonomic profiling based on capture-based data could achieve strain level resolution in the identification of microorganisms, which remains largely unfeasible with current shotgun and amplicon based approaches. For this, we used the bioinformatic software metaSNV to call for unique single nucleotide polymorphisms (SNP) in the ten marker genes that identified different strains in a sample.

As shown in table 1 below, the number of unique strains identified based on SNP detections under strict thresholds (i.e. SNPs supported by at least five reads and across various samples) were ten times larger using the capture-based targeted sequencing approach described in this document.

**Table 1**

| Exp. | **SNPs** | **Unique Strains** |
|---|---|---|
| ShotGun | 5.477 | 43 |
| Capture | 98.751 | 468 |

### DNA capture and sequencing

DNA from the different experiments was mechanically fragmented with Covaris (Covaris, Woburn, Massachusetts, USA) to an average fragment size of 300pb. Library preparation of the samples was done according to NEBNext Ultra II DNA Library Prep Kit with manufacturer's instructions. The samples were equimollary pooled, and captured using the Nimblegen baits, following the SeqCap EZ HyperCap Workflow v2.3. Illumina sequencing was performed afterwards, obtaining paired-end reads of 2x150 nucleotides. Shotgun data was already available for human gut samples, for extreme environment experiments and for soil samples.

### Reads preprocessing

Obtained Illumina reads were inspected with FastQC (v0.11.9) and MultiQC (v1.10.1). Duplicates were removed using BBmap's (v38.91-1) clumpify.sh (dedupe subs=0). Next, deduplicated reads were trimmed using fastp (v0.20.1; --disable_quality_filtering --disable_length_filtering --correction --trim_poly_g --overrepresentation_analysis) and Trimmomatic (0.38; ILLUMINACLIP:$EBRGOTTRIMMOMATIC/adapters/TruSeq3-PE.fa:2:30:10 MAXINFO:40:0.2 MINLEN:50). From the sets of clean reads we created standardized sets of read pairs of length 100, using Trimmomatic (v0.38; CROP:100 MINLEN:100). These were randomly subsampled to 1M read pairs for all samples, and also to the minimum number of reads available for each capture-shotgun sample pair.

Taxonomic classification of reads. We ran mOTUs version 3.0.1 with both -g1 and -g3, with the sets of standardized reads, with both subsampling to 1M read pairs and to the minimum of capture / shotgun sample pairs. For the latter we also ran mOTUs with - g5, -g7, -g9 and -g10. We also ran motus for all the available read pairs, both capture and shotgun data, with both -g 1 and -g 3, producing both absolute abundances from raw counts and relative abundances from scaled counts.

Saturation curves were built with mOTUs results (default parameters) for the samples with the largest number of standardized 2x100 read pairs for each environment (human gut: 16s001897, soil: NoOTCNoCrust3, extreme environments: LLA9C1), to compare capture and shotgun samples. Subsampling was performed in steps of 1M read pairs, reducing the number of replicates with the number of reads to be subsampled. For the human gut data, subsamples ranged 1M (5 replicates) to 7M read pairs (1 replicate). For soil, subsamples ranged from 1M (5 replicates) to 18M read pairs (1 replicate). For extreme environments, subsamples ranged 1M (5 replicates) to 13M read pairs (1 replicate).

We also used Kraken2. Some technical comments: most sequences are assigned with very low confidence thresholds (0.0, 0.02). No clear differences observed (which is somewhat surprising) analyzing the same number of reads from capture and shotgun data. In both cases, the kraken2 confidence parameter has dramatic impact in the number of species detected and % of reads mapped to all the taxonomic ranges, which are drastically decreased above confidence 0.2 (-1/5 of the k-mers of the read identified), specially for ext_env and soil. For human_gut, the number of species detected is much more affected than the % of reads mapped, by the confidence threshold. Overall, the diversity detected using Kraken2 is comparable, capture vs shotgun, but capture data is enriched in genes (COG MGs) which can be directly compared, in contrast with shotgun which will show a mixture of sequences from diverse genomic loci which will not directly compare.

Also, as it happens with motus, the kraken2 database seems biased towards human_gut diversity. We also ran MetaPhlAn version 4 with default parameters and unclassified fraction estimation.

### 16S sequencing and analysis

We obtained sequencing reads for 50 samples out of the 57 used for capture and shotgun data. PCR primers were removed with Cutadapt. Following dada2's pipeline, reads were trimmed, denoised, merged and subjected to chimera detection. Sample inference and ASV detection was carried out with dada2 default procedures. Taxonomic classification of ASVs was carried out with DECIPHER, both with the SILVA_SSU_r138_2019 and the GTDB_r207-mod_April2022 databases. We also used a GTDB custom database to classify the ASVs, as well as clustered ASVs to 97% identity, resembling a rather standard threshold for OTUs delineation.

### Detection of on-target reads

Clean reads were mapped to the original marker genes using Diamond (v2.0.11.149; blastx mode --ultra-sensitive --iterate -e 0.001 --matrix BLOSUM45 --top 3), and . Each read pair was kept, based on mOTUs BWA and Diamond hits, if at least one read of the pair maps to a marker gene, unless the other read maps to a different marker gene. The remaining read pairs were mapped to eggNOG 5 with eggNOG-mapper 2.1.6 (-m diamond --sensmode ultra-sensitive --dmnd_iterate yes -matrix BLOSUM45). If at least one read of each pair mapped to an OG different from the marker genes, the read pair was discarded. The remaining read pairs were classified as on-target.

## Claims

1. An *in vitro* method for providing a collection of sequencing probes that enable targeting, preferably in a single sequencing experiment, one or various gene families or functions with full phylogenetic coverage and high sensitivity, said method comprising the steps of:
a. Step 1. Gene family selection: Compiling a list of marker gene families or functional orthologous groups (OG) by, preferably, identifying genes from a database within or associated to a specified group of molecular functions.
b. Step 2 Data mining: Identifying and providing from one or more databases, a data corpus or dataset of nucleotide sequences having a percentage of identity with the genes and OGs identified in step 1 of at least 60%, 70%, 80%, 90%, 95%, 99% or more;
c. Step 3. Design of capture-kits: Analyzing the data corpus or dataset provided in Step 2 in order to reduce its dimensionality to make it accessible for a single targeted sequencing experiment, preferably while maintaining the same phylogenetic coverage and sensibility.
d. Providing the design of the panel or collection of sequencing probes that enable targeting, preferably in a single sequencing experiment, one or various gene families or functions selected in Step 1.
e. Optionally, manufacturing or producing the panel of step d.

2. The method according to claim 1, wherein in Step 2 the data corpus of nucleotide sequences is provided by retrieving for each of the selected OGs of step 1, the sequences from (i) global genomic repositories and (ii) public metagenomic datasets.

3. The method according to claim 2, wherein Step 3 is carried out by a method comprising the following steps i) to iv) for each targeted OG (tOG) included in a kit:
i. Decomposing into non-overlapping chunks of from 10 - 200 pair bases, preferably from 50 to 100 pair bases, more preferably about 80 pair bases, all or part of the nucleotide sequences comprising the dataset obtained in Step 2 for each of the tOG, herein referred to as probes.
ii. Aligning the nucleotide sequences comprising the dataset obtained in step 2 into a multiple sequence alignment (MSA), where the conservation level of each column is calculated.
iii. The multiple sequence alignment produced is used to generate a phylogenetic tree of all or part of the non-redundant sequences.
iv. All or part of the generated probes in step i) are compared in an all-against-all fashion to calculate their pairwise sequence similarity, computed as the % of nucleotides identities observed between two probes and provide an all-against-all probe comparison matrix.
wherein the method further comprises the following steps:
v. Using the all-against-all probe comparison matrix of step iv to generate clusters of probes at the NSI threshold, wherein the NSI threshold is selected from one or more values in the range of from 30% to 100%.
vi. Mapping all or part of the probe clusters of step v back to the same original genomic and metagenomic databases used in Step 2 using BLASTN searches.
vii. Analyzing to compute the ratio of positive matches (i.e. hits against a gene belonging to the expected OG) over negative matches (i.e. hits against a gene belonging to an unwanted OG) of the hits produced in step vi.
viii. Discarding all probe clusters with a ratio of positive matches lower than 1.0 (i.e. probes producing at least one mismatch) from the set.
ix. Mapping all or part of the remaining selected probe clusters to the MSA generated in step ii), preferably by recording the exact position of matching residues of the original probe on the MSA.
x. Discarding probes matching columns of the alignment produced in ii with a residue conservation score, preferably of below 0.75.
xi. Mapping back the final set of selected probes to the original sequences selected in Step 2, preferably by using BLASTN, and preferably calculating: number of original sequences recovered by the probes (RecovT), the average length of the original sequence covered by the probe-selection (RecovLen) and the phylogenetic coveraged based on the the generated in substep C (PhyloC)

4. The method according to claim 3, wherein for each tOG the total number of probes selected (Nprobes), together with their RecovT, RecovL and PhyloC values obtained for each clustering experiment under each NSI are automatically evaluated to select the set of probes that maximizes RecovT, RecovL and PhyloC, while minimizing NProbes.

5. The method according to claim 4, wherein the set of probes that maximizes RecovT, RecovL and PhyloC, while minimizing Nprobes, for each tOG, is combined into a single dataset, and providing the design of the panel.

6. The method according to claim 5, wherein the method further comprises the manufacturing or production of the panel of probes.

7. A panel obtained or manufactured or produced by the method of claim 6, wherein the probes are polynucleotides, preferably DNA or RNA, probes.

8. Use of the panel according to claim 6, for the capture and target enrichment assays directly on environmental DNA samples, prior to shotgun sequencing.

9. A kit comprising the panel obtained or manufactured or produced by the method of claim 6
